# EUROPÄISCHE PATENTSCHRIFT

(11) **EP 1 834 597 B1**
(45) Veröffentlichungstag und Bekanntmachung des Hinweises auf die Patenterteilung: **26.08.2015**
(21) Anmeldenummer: 06004822.0
(22) Anmeldetag: 09.03.2006
(51) Int. Cl.: A61B 17/88

(54) **Kraftwirkungs-Rückmeldung bei chirurgischen Instrumenten**
Surgical instrument comprising load feedback
Dispositif chirurgical avec rétroaction de la charge

(43) Veröffentlichungstag der Anmeldung: 19.09.2007
(73) Patentinhaber: Brainlab AG, 85622 Feldkirchen (DE)
(72) Erfinder: Neubauer, Timo, 85586 Poing/ Angelbrechting (DE); Saffari, Anusch, 83666 Waakirchen (DE); Millahn, Manuel, 80799 München (DE)
(74) Vertreter: Schwabe - Sandmair - Marx

(56) Entgegenhaltungen:
- WO-A-02/36018
- WO-A-03/101322
- WO-A2-02/07617
- DE-U1- 20 015 842
- US-A1- 2004 220 602

## Beschreibung

Die Erfindung betrifft die Kraftwirkungs-Rückmeldung bei chirurgischen Instrumenten. Insbesondere betrifft sie ein chirurgisches Instrument mit einem Halteabschnitt und einem an dem Halteabschnitt anbringbarem Werkzeug, sowie ein medizintechnisches Instrumentenüberwachungssystem mit einem solchen chirurgischen Instrument.

Ein Beispiel für ein solches chirurgisches Instrument ist eine Knochensäge. Bei solchen Knochensägen kommt es vor, dass sich das Sägeblatt deformiert, weil eine zu hohe Last ausgeübt wird, während ein sehr widerstandsfähiges Material zersägt wird. Dies kann den Schneidvorgang selbst beeinträchtigen, aber auch zu unregelmäßigen Schnittflächen führen. Wenn das Instrument im Rahmen einer chirurgischen Navigation überwacht wird, führen Verformungen des Werkzeugs nachteiligerweise auch zu Navigationsfehlern.

In der DE 100 24 221 D1 wird für eine chirurgische Säge ein Lösungsvorschlag für die oben angesprochenen Probleme gemacht. Gemäß diesem Vorschlag soll an oder in dem Sägeblatt ein Biege- und Positionssensor angeordnet werden, der seiner räumlichen Konfiguration entsprechende Signale erzeugt und diese einer Signalverarbeitungseinrichtung zuführt. Ein Problem bei diesem Ansatz steht darin, dass jedes Werkzeug, d.h. jedes Sägeblatt selbst mit einer solchen Sensoreinrichtung versehen werden muss. Dies macht die Sägeblätter, die der Abnutzung unterliegen, teuer in Anschaffung und Herstellung. Außerdem sind solchermaßen mit Sensoren versehene Sägeblätter aufwändig zu sterilisieren.

Es ist die Aufgabe der vorliegenden Erfindung, ein chirurgisches Instrument mit einem an einem Halteabschnitt anbringbaren Werkzeug so auszugestalten, dass die oben beschriebenen Nachteile nicht mehr auftreten, aber trotzdem eine Lasterfassung möglich wird. Insbesondere soll auch ein Instrument bereitgestellt werden, das mit herkömmlichen Werkzeugen arbeiten kann, die günstig angeschafft werden können und einfach sterilisierbar sind.

Diese Aufgabe wird durch ein chirurgisches Instrument gemäß dem Anspruch 1 gelöst. Die Unteransprüche definieren bevorzugte Ausführungsformen der Erfindung. Gemäß der vorliegenden Erfindung weist das Instrument mindestens einen Lastsensor auf, der die mechanische Last misst, die das Werkzeug am Instrument ausübt. Mit anderen Worten wird nicht mehr direkt gemessen, wie das Werkzeug selbst sich verformt, sondern die Lastmessung wird an einen Punkt verlagert, an dem festgestellt werden kann, welche Last das Werkzeug auf das Instrument überträgt. Die Last, die der Bediener des chirurgischen Instruments auf das Werkzeug ausübt, setzt sich vom Werkzeug selbst durch das Instrument hindurch fort, und diesen Umstand nützt die vorliegende Erfindung, um das Messmittel, also den Lastsensor vom Werkzeug selbst weg und in einen beliebigen anderen Teil des chirurgischen Instruments zu setzen. Hierdurch wird es wieder möglich, herkömmliche Instrumente zu verwenden, wie sie auf dem Markt erhältlich sind und welche in üblicher und einfacher Weise sterilisiert werden können. Für diese Werkzeuge lassen sich zulässige Lasten ermitteln.

Der Lastsensor kann einen Kraft- oder Momentensensor umfassen, und solche Sensoren können zur erfindungsgemäßen Verwendung angepasst werden.

Erfindungsgemäss ist der Lastsensor mit einer Lastanzeige am Instrument, insbesondere am Halteabschnitt, gekoppelt, um die auftretende Last zur Überwachung anzuzeigen. Eine solche Lastanzeige kann eine oder mehrere Lastzustandsanzeigen aufweisen, die den Lastzustand, insbesondere farblich codiert, wiedergeben.

Bei einer Ausführungsvariante weist das Instrument einen Werkzeugadapter auf, insbesondere eine Universaladapter für eine Vielzahl passender Werkzeuge, der zum Beispiel als Steck- oder Rastverbindung ausgestaltet ist. Der Lastsensor kann dann auch für unterschiedliche Werkzeuge die mechanische Last messen, so dass der Lastzustand immer angezeigt werden kann.

Es besteht erfindungsgemäß die Möglichkeit, das Werkzeug mit einem Identifikationsmittel zu versehen, das einer Gegenstelle am Instrument, insbesondere am Halteabschnitt, mechanisch oder drahtlos eine Information über die Natur des Werkzeugs übermittelt. Das Identifikationsmittel kann bei einer solchen Ausführungsform ein RFID (Radio Frequency Identification = Hochfrequenz-Identifikation)-Transponder sein, wobei die Gegenstelle ein RFID-Empfänger bzw. eine RFID-Sender/Empfängereinheit ist.

Mit der Identifizierungstechnik lassen sich nun auch werkzeugspezifische Lastzustände messen und anzeigen. Man ermittelt beispielsweise für ein bestimmtes Werkzeug vorab, welche Last erlaubt ist, ohne dass sich am Werkzeug Verformungen zeigen oder die Funktion in sonstiger Art und Weise beeinträchtigt wird. Wenn man die Lastanzeige nun für spezielle Werkzeuge in geeigneter Weise anpasst, kann für jedes Werkzeug beispielsweise angegeben werden, ob es im zulässigen Lastbereich benutzt wird oder ob eine Überlastung auftritt.

Das chirurgische Instrument welches einen Werkzeugantrieb aufweist, ist ein sogenanntes "power tool".

Der Lastsensor ist an einer Stelle im Antriebsstrang des Werkzeugs angeordnet.

Ein Ausführungsbeispiel des erfindungsgemäßen chirurgischen Instruments ist eine chirurgische, elektrisch oder pneumatisch betriebene Säge, insbesondere eine Knochensäge mit einem oszillierenden Sägeblattwerkzeug. Es ist dabei anzumerken, dass die Erfindung grundsätzlich bei allen chirurgischen Instrumenten anwendbar ist, bei denen Lasten über ein Werkzeug ausgeübt werden und bei denen es von Vorteil ist, diese Lasten zu überwachen.

Gemäß einem weiteren Aspekt betrifft die vorliegende Erfindung ein medizintechnisches Instrumentenüberwachungssystem mit einem chirurgischen Instrument, wie es in verschiedenen Ausführungsformen oben beschrieben worden ist. Das Instrument weist dabei eine Übertragungseinheit auf, die den vom Lastsensor ermittelten Lastzustand zu einem Empfänger überträgt, der einer Datenverarbeitungseinheit zugeordnet ist. Mit anderen Worten wird gemäß dem erfindungsgemäßen Instrumentenüberwachungssystem der Lastzustand weitergegeben und verarbeitet, was bei verschiedenen Ausführungen von Vorteil sein kann, beispielsweise bei einer Variante, bei der die Datenverarbeitungseinheit eine Bildausgabe umfasst, auf welcher der Lastzustand ausgegeben werden kann. Oftmals ist es von Vorteil, wenn die Ausgabe des Lastzustands nicht am Instrument selbst erfolgt, sondern größer und deutlicher auf einer separaten Bildausgabe durchgeführt wird, z.B. auf einem Überwachungs- oder Navigationsbildschirm im Operationsraum. Zusätzlich können natürlich auch akkustische Signale ausgegeben werden, die einen Überlastzustand hörbar anzeigen.

Die Datenverarbeitungseinheit kann einem medizintechnischen Tracking- und Navigationssystem zugeordnet sein, welches das Instrument positionell bestimmt und verfolgt In diesem Fall wird das Tracking- bzw. Navigationssystem vorteilhafterweise eine Auswertungseinheit umfassen, die den Lastzustand und die Bewegung des Instruments korreliert und Daten über notwendige oder mögliche Anpassungen der Bewegungen oder der Bedienung ermittelt, die insbesondere über die Bildausgabe ausgegeben werden.

Die vorliegende Erfindung verbessert und vereinfacht chirurgische Verfahren, die mit lastsensitiven Werkzeugen durchgeführt werden, beispielsweise das Sägen von Knochen bei Total-Knieersetzungen. Wenn die Anzeige und die Identifikationsmerkmale umgesetzt werden, kann das System aufgrund der Informationen, das es beispielsweise aus dem Transponder erhält (wie z.B. Material- und Geometriedaten) die Maximalkraft bestimmen, die gestattet ist, ohne dass sich das Werkzeug verformt. Die Information des Sensors kann mit der Information aus dem Navigationssystem kombiniert werden, um dem Verwender eine präzise Anzeige über die Tiefe des Werkzeugeingriffes zu vermitteln, und dies kann auch dazu verwendet werden, die Geschwindigkeit bzw. Drehzahl des Werkzeugs an die Dichte des Gewebes anzupassen, das gerade behandelt wird.

Mit der Erfindung erhält der Chirurg intraoperative Informationen über die Kräfte, die auf das Werkzeug wirken. Diese Rückmeldung (Feedback) gibt dem Chirurgen die Möglichkeit, einen zu hohen Druck auf das Instrument und damit auf das Risiko der Deformierung des Werkzeugs zu reduzieren. Es wird z.B. möglich, einen Knochen präziser zu bearbeiten, so dass Implantate wie z.B. Knieersatz-Implantate besser passen. Wenn ein Transponder-System (RFID) verwendet wird, kann die Software werkzeugspezifische Informationen (wie geometrische Daten) automatisch ermitteln. Dies vermindert das Risiko von Kalibrierungsfehlern und macht die Verwendung navigierter Instrumente leichter. Ein navigiertes chirurgisches Instrument gemäß der vorliegenden Erfindung stellt präzise Daten über die Eingriffstiefe bereit, und auch diese Daten können verwendet werden, um beispielsweise den Antrieb (Geschwindigkeit, Drehzahl) des Werkzeugs auf die Dichte des Gewebes einzustellen, das gerade behandelt wird oder dessen Behandlung bevorsteht.

Die Erfindung wird im Weiteren anhand von Ausführungsformen näher erläutert. Sie kann alle hierin beschriebenen Merkmale einzeln und in jedweder Kombination umfassen. In den Zeichnungen zeigen:
- Figur 1: ein erfindungsgemäßes chirurgisches Instrument, ausgestaltet als chirurgische Säge, im Navigationsumfeld;
- Figur 2: ein Detail des chirurgische Instruments mit einer Lastsensoranordnung;
- Figur 3: einen Abschnitt einer Sägeaufsatz-Kupplung mit Verkippungs-Messung;
- Figur 4: einen Ausschnitt einer Bohraufsatz-Kupplung mit Axialkraft-Messung; und
- Figur 5: einen Ausschnitt einer Sägeaufsatz-Kupplung mit Kraftmessung in der Einspannung.

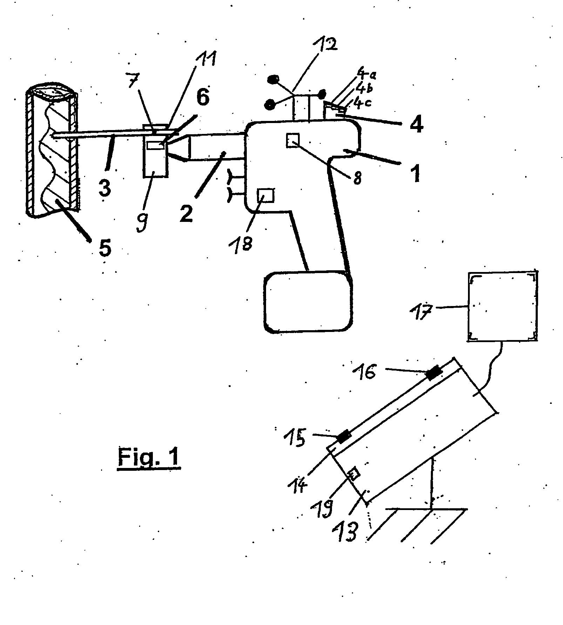

In der Figur 1 zeigt das Bezugszeichen 1 auf den Halteabschnitt bzw. den Handgriff einer chirurgischen Säge mit einer Drehkupplung 2, an der vorne der Werkzeughalter 9 angebracht ist. Der Werkzeughalter 9 oszilliert drehend, und er weist einen Werkzeugadapter 11 auf, in den das Sägeblatt 3 der Steckverbindung eingesetzt ist. Das Sägeblatt 3 hat einen RFID-Transponder 7, der Informationen über das Werkzeug, d.h. das Sägeblatt 3, an den RFID-Sender/Empfänger 8 übermittelt. Damit wird das Werkzeug 3 eindeutig identifiziert, und zwar bezüglich seiner körperlichen Merkmale, wie z.B. der Länge bzw. der vorderen Position des funktionellen Abschnittes, also der Teil des Sägeblattes, der tatsächlich schneidet. Es werden aber auch Daten über anderen Merkmale des Sägeblatts 3 übertragen, beispielsweise über die zulässige Last, die beim Sägevorgang auftreten darf, ohne dass sich das Sägeblatt 3 deformiert.

Die tatsächliche auftretende Last wird durch einen am Werkzeughalter 9 angebrachten Lastsensor 6 (Kraft- bzw. Momentensensor) ermittelt, nämlich die Last, die das Sägeblatt 3 z.B. auf den Werkzeughalter 9 ausübt. Diese festgestellte Last kann nunmehr mit Hilfe einer nicht dargestellten Datenübertragen (per Kabel oder kabellos) an eine Datenverarbeitungseinheit (z.B. im Halteabschnitt) übertragen werden, welche den Lastzustand mit den zulässigen Lastzuständen korreliert, und dann kann ein Signal ausgegeben werden, welches dem Verwender den Lastzustand anzeigt.

Ein Weg der Ausgabe eines Signals ist die Verwendung einer Lastzustandsanzeige 4, die im dargestellten Ausführungsbeispiel drei LEDs 4a, 4b und 4c umfasst. Es bieten sich dabei verschiedene Möglichkeiten, beispielsweise kann eine Überlastung vorhanden sein, wenn alle drei LEDs aufleuchten, wobei die LEDs aber auch farblich codiert sein können, z.B. grün, gelb und rot für einen normalen, erhöhten und einen Überlastzustand.

Der Verwender kann dann seine Kraftausübung beim Sägen eines Knochens 5, der eine härtere Außenschicht und einen weicheren Kern aufweist, je nach gemeldetem Lastzustand anpassen.

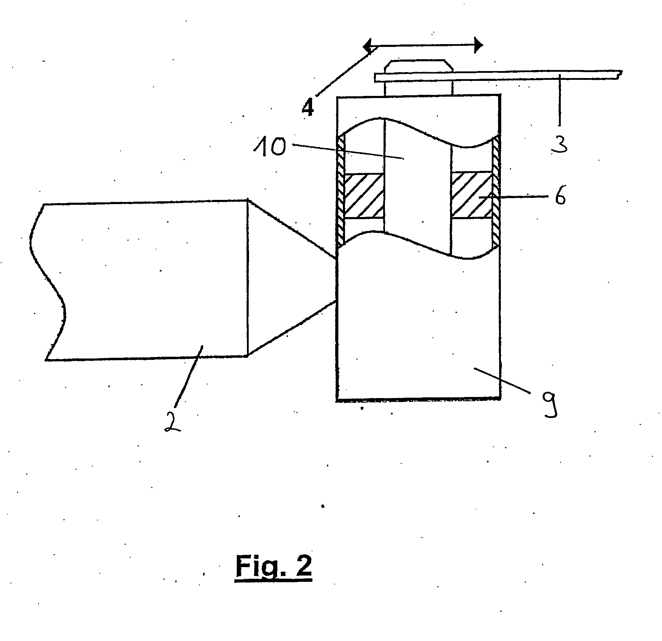

In der Figur 2 ist noch eine abgewandelte Ausführungsform etwas detaillierter dargestellt, bei der das Sägeblatt 3 auf das Ende einer oszillierenden Übertragungswelle 10 aufgesteckt und dort gesichert ist. Der Lastsensor 6 befindet sich am Werkzeughalter 9, der auf der Kupplung 2 aufgesetzt ist, und zwar an einer etwas erhöhten Stelle, und er ist mit der Welle 10 im Eingriff. Je nachdem, wo der Lastsensor angeordnet ist, kann er größere oder kleinere Kräfte bzw. Momente aufnehmen und weitermelden. Bei der in Figur 2 dargestellten Ausführungsform wird mit dem Sensor 6 die Verkippung der oszillierenden Welle 10 gemessen, die durch den Pfeil 4 angedeutet ist. Der Lastsensor könnte aber grundsätzlich auch in der Drehkupplung 2 oder in daran anschließenden Bauteilen vorgesehen werden, je nachdem wie direkt die Lastmessung sein muss.

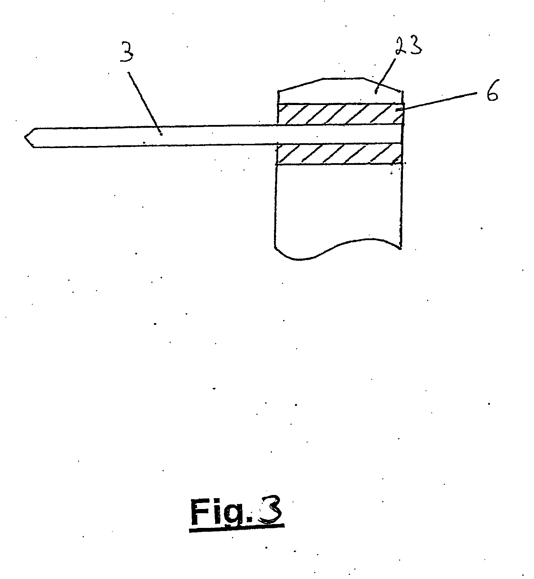

Weitere Anordnungsmöglichkeiten für den Lastsensor zeigt die Figur. Die Figur 3 zeigt schließlich eine weitere Messmethode, bei der ein Sensor 6 in einer Sägeblatt-Halterung 23 untergebracht ist, wobei das Sägeblatt 3 direkt durch den Sensor 6 gehalten wird, der in dieser Weise die Kraft messen kann, die in der Einspannung des Sägeblattes 3 auftritt. Natürlich ist auch eine solche Ausführungsform, bei der der Sensor selbst als Einspannung eines Werkzeuges dient, ein Teil der vorliegenden Erfindung.

Wenn man sich nun wieder der Figur 1 zuwendet, ist zu sehen, dass die dort dargestellte Knochensäge in einem chirurgischem Navigationsumfeld verwendet wird. Sie weist dazu den optisch erfassbaren Referenzstern 12 auf, dessen Position von einem Navigations-/Trackingsystem ermittelt werden kann, das unten links dargestellt ist. Es umfasst einen Tracking/Kameraeinheit 14, mit Kameras 15, 16, welche die Position des chirurgischen Instruments über den Referenzstern 12 ermitteln und damit auch die Position des Sägeblattes 3 bzw. seiner Spitze. Dies ist insbesondere dann möglich, wenn das chirurgische Instrument mit seinen Daten und auch mit den Informationen über die körperlichen Merkmale der Säge 3 im Speicher des Navigationssystems hinterlegt ist, das mit dem Bezugszeichen 13 bezeichnet ist (Vorkalibrierung bzw. vorkalibriertes Instrument).

Eine Aufgabe des Navigationssystems 13 ist es also, das chirurgische Instrument, d.h. die chirurgische Säge positionell zu verfolgen, diese Positionsdaten mit vorab ermittelten Körperstrukturdaten (CT, MR) zu korrelieren und dem behandelnden über den Bildschirm 17 eine bildunterstützte Navigation zur Verfügung zu stellen.

Im Zusammenhang mit der vorliegenden Erfindung kann das Navigationssystem aber auch weitere Aufgabe erfüllen. So kann die chirurgische Säge beispielsweise über einen Sender 18 Informationen über den Lastzustand am Sägeblatt 3 übermitteln, die von einem Empfänger 19 am Navigationssystem 13 empfangen werden. Diese Informationen über den Lastzustand lassen sich dann in der Datenverarbeitung des Navigationssystems verarbeiten, und mittels des Bildschirms 17 können auf dem Lastzustand beruhende unterstützende Informationen ausgegeben werden. Diese Informationen können Anweisungen sein, die Kraft auf die Säge, d.h. auch auf das Sägeblatt 3, zu vermindern oder die Schwingungszahl herabzusetzen. Es kann auch angezeigt werden, dass im weiteren Verlauf ein Knochengewebe bearbeitet werden wird, das eine geringere oder eine höhere Werkzeugkraft erfordert. Weil die Last immer so kontrolliert werden kann, dass das Sägeblatt 3 sich nicht verbiegen wird, kann man wiederum davon ausgehen, dass der funktionelle Abschnitt des Sägeblattes, nämlich dessen sägender vorderer Abschnitt, sich tatsächlich an der Stelle befindet, die vom Navigationssystem angezeigt wird. All diese Überwachungs- und Kontroll- bzw. Rückmeldungsfunktionen gestatten eine präzise Behandlung und dienen damit dem Wohl des Patienten.

## Patentansprüche

1. Chirurgisches Powertool mit einem Halteabschnitt (1) und einem an dem Halteabschnitt (1) anbringbaren Werkzeug (3), wobei das Powertool mindestens einen Lastsensor (6) aufweist, der die mechanische Last misst, die das Werkzeug (3) am Powertool ausübt, wobei das Powertool einen Werkzeugantrieb aufweist und der Lastsensor (6) an einer Stelle im Antriebsstrang des Werkzeugs (3) angeordnet ist, **dadurch gekennzeichnet, dass** der Lastsensor (6) mit einer Lastanzeige (4) am Powertool, insbesondere am Halteabschnitt, gekoppelt ist.

2. Powertool nach Anspruch 1, **dadurch gekennzeichnet, dass** der Lastsensor (6) einen Kraft- oder Momentensensor umfasst.

3. Powertool nach einem der Ansprüche 1 bis 2, **dadurch gekennzeichnet, dass** die Lastanzeige (4) eine oder mehrere Lastzustandsanzeigen (4a, 4b, 4c) aufweist, die den Lastzustand, insbesondere farblich codiert, wiedergeben.

4. Powertool nach einem der Ansprüche 1 bis 3, **dadurch gekennzeichnet, dass** es einen Werkzeugadapter (11) aufweist, insbesondere einen Universaladapter für eine Vielzahl passender Werkzeuge, der speziell als Steck- oder Rastverbindung ausgestaltet ist.

5. Powertool nach einem der Ansprüche 1 bis 4, **dadurch gekennzeichnet, dass** das Werkzeug (3) ein Identifikationsmittel (7) aufweist, das einer Gegenstelle (8) am Powertool, insbesondere am Halteabschnitt (1), mechanisch oder drahtlos eine Information über die Natur des Werkzeugs (3) übermittelt.

6. Powertool nach Anspruch 5, **dadurch gekennzeichnet, dass** das Identifikationsmittel ein RFID (Radio Frequency IDentification = Hochfrequenz-Identifikation)-Transponder und die Gegenstelle ein RFID-Empfänger bzw. eine RFID-Sender/Empfängereinheit ist.

7. Powertool nach einem der Ansprüche 1 bis 6, **dadurch gekennzeichnet, dass** der Lastsensor (6) sich an dem Werkzeugadapter (11) oder an einem den Werkzeugadapter umfassenden Antriebsglied (6) befindet.

8. Powertool nach einem der Ansprüche 1 bis 6, **dadurch gekennzeichnet, dass** am Halteabschnitt (1) eine antriebsübertragende Kupplung (2) angeordnet ist und der Lastsensor (6) sich an der Kupplung befindet.

9. Powertool nach einem der Ansprüche 1 bis 8, **dadurch gekennzeichnet, dass** es eine chirurgische, elektrisch oder pneumatisch betriebene Säge, insbesondere eine Knochensäge mit einem oszillierenden Sägeblattwerkzeug ist.

10. Medizintechnisches Instrumentenüberwachungssystem mit einem chirurgischen Powertool nach einem der Ansprüche 1 bis 9, **dadurch gekennzeichnet, dass** das Powertool eine Übertragungseinheit (18) aufweist, die den vom Lastsensor (6) ermittelten Lastzustand zu einem Empfänger (19) überträgt, der einer Datenverarbeitungseinheit zugeordnet ist.

11. System nach Anspruch 10, **dadurch gekennzeichnet, dass** die Datenverarbeitungseinheit eine Bildausgabe (17) umfasst, auf welcher der Lastzustand ausgegeben werden kann.

12. System nach Anspruch 10 oder 11, **dadurch gekennzeichnet, dass** die Datenverarbeitungseinheit einem medizintechnischen Tracking- bzw. Navigationssystem (14, 13) zugeordnet ist, welches das Powertool positionell bestimmt und verfolgt.

13. System nach Anspruch 12, **dadurch gekennzeichnet, dass** das Tracking- bzw. Navigationssystem (14, 13) eine Auswertungseinheit umfasst, die den Lastzustand und die Bewegung des Powertools korreliert und Daten über notwendige oder mögliche Anpassungen der Bewegung oder der Bedienung ermittelt, die insbesondere über die Bildausgabe (17) ausgegeben werden.

## Claims

1. A surgical power tool comprising a holding section (1) and a tool (3) which can be attached to the holding section (1), wherein the power tool comprises at least one load sensor (6) which measures the mechanical load which the tool (3) on the power tool exerts, wherein the power tool comprises a tool drive and the load sensor (6) is arranged at a point in the drive chain of the tool (3), **characterised in that** the load sensor (6) is coupled to a load indicator (4) on the power tool, in particular on the holding section.

2. The power tool according to Claim 1, **characterised in that** the load sensor (6) includes a force or torque sensor.

3. The power tool according to any one of Claims 1 to 2, **characterised in that** the load indicator (4) comprises one or more load state indicators (4a, 4b, 4c) which indicate the load state, in particular by a colour code.

4. The power tool according to any one of Claims 1 to 3, **characterised in that** it comprises a tool adaptor (11), in particular a universal adaptor for a number of matching tools, which is specifically embodied as a plug or latch connection.

5. The power tool according to any one of Claims 1 to 4, **characterised in that** the tool (3) comprises an identification means (7) which mechanically or wirelessly transmits information about the nature of the tool (3) to a receiver (8) on the power tool, in particular on the holding section (1).

6. The power tool according to Claim 5, **characterised in that** the identification means is an RFID (radio-frequency identification) transponder and the receiver is an RFID receiver or an RFID transmitter/receiver unit.

7. The power tool according to any one of Claims 1 to 6, **characterised in that** the load sensor (6) is situated on the tool adaptor (11) or on a drive member (6) which includes the tool adaptor.

8. The power tool according to any one of Claims 1 to 6, **characterised in that** a drive-transmitting coupling (2) is arranged on the holding section (1) and the load sensor (6) is situated on the coupling.

9. The power tool according to any one of Claims 1 to 8, **characterised in that** it is a surgical, electrically or pneumatically operated saw, in particular a bone saw, comprising an oscillating saw blade tool.

10. A medical instrument monitoring system comprising a surgical power tool according to any one of Claims 1 to 9, **characterised in that** the power tool comprises a transmission unit (18) which transmits the load state ascertained by the load sensor (6) to a receiver (19) which is assigned to a data processing unit.

11. The system according to Claim 10, **characterised in that** the data processing unit includes an image output (17) on which the load state can be outputted.

12. The system according to Claim 10 or 11, **characterised in that** the data processing unit is assigned to a medical tracking and/or navigation system (14, 13) which determines and tracks the position of the power tool.

13. The system according to Claim 12, **characterised in that** the tracking and/or navigation system (14, 13) includes an evaluation unit which correlates the load state and the movement of the power tool and ascertains data about necessary or possible adjustments to movement or operation, which are in particular outputted via the image output (17).

## Revendications

1. Outil électrique chirurgical avec une partie support (1) et un outil (3) apte à être fixé sur la partie support (1), où l'outil électrique comporte au moins un capteur de charge (6) mesurant la charge mécanique exercée par l'outil (3) sur l'outil électrique, où l'outil électrique comporte un entraînement d'outil et le capteur de charge (6) est disposé en un point de la transmission de l'outil (3), **caractérisé en ce que** le capteur de charge (6) est couplé avec un affichage de charge (4) sur l'outil électrique, notamment sur la partie support.

2. Outil électrique selon la revendication 1, **caractérisé en ce que** le capteur de charge (6) comprend un capteur de force ou un capteur de moment.

3. Outil électrique selon l'une des revendications 1 à 2, **caractérisé en ce que** l'affichage de charge (4) comporte un ou plusieurs affichages d'état de charge (4a, 4b, 4c) qui représentent l'état de charge, notamment via un code couleur.

4. Outil électrique selon l'une des revendications 1 à 3, **caractérisé en ce qu'**il comporte un adaptateur d'outil (11), notamment un adaptateur universel pour une pluralité d'outils adaptés, spécifiquement configuré comme un connecteur enfichable ou un connecteur d'encliquetage.

5. Outil électrique selon l'une des revendications 1 à 4, **caractérisé en ce que** l'outil (3) comporte un moyen d'identification (7) qui communique, mécaniquement ou sans fil, une information sur la nature de l'outil (3) à un point de destination (8) disposé sur l'outil électrique, notamment sur la partie support (1).

6. Outil électrique selon la revendication 5, **caractérisé en ce que** le moyen d'identification est un transpondeur RFID (Radio Frequency IDentification = radio-identification) et le point de destination est un récepteur RFID ou une unité d'émission/réception RFID.

7. Outil électrique selon l'une des revendications 1 à 6, **caractérisé en ce que** le capteur de charge (6) se trouve sur l'adaptateur d'outil (11) ou sur un organe d'entraînement (6) comprenant l'adaptateur d'outil.

8. Outil électrique selon l'une des revendications 1 à 6, **caractérisé en ce qu'**un accouplement (2) transmettant l'entraînement est disposé sur la partie support (1) et que le capteur de charge (6) se trouve sur l'accouplement.

9. Outil électrique selon l'une des revendications 1 à 8, **caractérisé en ce qu'**il consiste en une scie chirurgicale électrique ou pneumatique, en particulier une scie à os avec un outil de lame de scie oscillante.

10. Système de surveillance d'instrument médico-technique avec un outil électrique chirurgical selon l'une des revendications 1 à 9, **caractérisé en ce que** l'outil électrique comporte une unité de transmission (18) qui transmet l'état de charge déterminé par le capteur de charge (6) à un récepteur (19) associé à une unité de traitement de données.

11. Système selon la revendication 10, **caractérisé en ce que** l'unité de traitement de données comprend un affichage (17) sur lequel peut être affiché l'état de charge.

12. Système selon la revendication 10 ou 11, **caractérisé en ce que** l'unité de traitement de données est associée à un système de navigation et/ou de localisation médico-technique (14, 13) déterminant et suivant la position de l'outil électrique.

13. Système selon la revendication 12, **caractérisé en ce que** le système de navigation et/ou de localisation (14, 13) comporte une unité d'évaluation qui établit une corrélation entre l'état de charge et le mouvement de l'outil électrique et calcule des données concernant des ajustements nécessaires ou possibles du mouvement ou du maniement, qui sont affichées en particulier via l'affichage (17).
